## Europäisches Patentamt

## European Patent Office

## Office européen des brevets

(11) Veröffentlichungsnummer: **0 467 362 A1**

# (12) EUROPÄISCHE PATENTANMELDUNG

(21) Anmeldenummer: **91112026.9**

(22) Anmeldetag: **18.07.91**

(51) Int. Cl.5: **A61M 16/08**

(30) Priorität: **20.07.90 DE 4023108**

(43) Veröffentlichungstag der Anmeldung:
**22.01.92 Patentblatt 92/04**

(84) Benannte Vertragsstaaten:
**AT CH DE FR GB IT LI NL SE**

(71) Anmelder: **EMS GmbH**
**Waldstrasse 2**
**W-8520 Möhrendorf(DE)**

(72) Erfinder: **Scmidt Horst**
**Am unteren See 12**
**W-8710 Kitzingen(DE)**

(74) Vertreter: **Hafner, Dieter et al**
**Dipl.-Phys. Dr. D. Hafner - Dipl.-Ing. H. Stippl**
**Ostendstrasse 132**
**W-8500 Nürnberg 30(DE)**

(54) **Beatmungsbesteck.**

(57) Die vorliegende Erfindung betrifft ein Beatmungsbesteck zur manuellen oder maschinellen Beatmung oder zur spontanen Atmung. Das Beatmungsbesteck weist einen Schlauch zur Zuführung von Narkosegasen (Frischgasschlauch) einen Faltenschlauch zur Abführung von Expirationsgasmengen und ggf. nicht eingeatmetem Gas (Expirationsgasschlauch) einen Beatmungsbeutel, welcher im Exprirationsgasweg angeordnet ist, eine Beatmungsmaske sowie ein Verbindungsstück von Frischgasschlauch und Expirationsgasschlauch an der Beatmungsmaske auf. Zur Erhöhung der sog. Resistance oder Complyance des Beatmungsbestecks sowie zur Erhöhung der Patientensicherheit vor Druckerhöhungen ist erfindungsgemäß an dem dem Patienten abgewendeten Ende des Expirationsgasschlauchs (4) ein Ventil (6) vorgesehen, welches den Expirationsgasschlauch mit einer Absaugung oder Entsorgung verbindet und die Aufrechterhaltung eines gewissen Druckniveaus im Beatmungskreis gewährleistet. Weiterhin ist vorgesehen, daß der Beatmungsbeutel (5) über eine Verbindungseinrichtung zwischen Beatmungsmaske (3) bzw. Endotracheal-Katheder und Ventil (6) in den Expirationsgasschlauch (4) eingeschaltet ist.

EP 0 467 362 A1

FIG. 1

Die Erfindung betrifft ein Beatmungsbesteck zur manuellen oder maschinellen Beatmung oder spontanen Atmung, insbesondere Narkosebeatmung mit einem Schlauch zur Zuführung von Gasen, insbesondere Narkosegasen (Frischgasschlauch), einem Schlauch, vorzugsweise Faltenschlauch zur Abführung von Expirationsgasmengen und ggf. nicht eingeatmetem Gas (Expirationsgasschlauch), einem Beatmungsbeutel, welcher im Expirationsgasweg angeordnet ist, einer Beatmungsmaske oder Endotrachealkatheter sowie einem Verbindungsstück zur Verbindung von Frischgasschlauch und Expirationsgasschlauch an der Beatmungsmaske bzw. am Endotrachealkatheter.

Beatmungsbestecke werden regelmäßig zur Durchführung von Inhalationsnarkosen insbesondere bei Kindern sowie für die Reanimation bzw. Beatmung, z. B im Kreissaal oder in Intensivstationen aber auch zur Reanimation oder Aufrechterhaltung einer Beatmung während des Transports, bei Geräteausfall oder Gerätewechsel verwendet. Die Beatmung erfolgt hierbei über eine Beatmungsmaske oder einen Tubus, insbesondere Endotrachealkatheter. Das Beatmungsbesteck wird je nach Einsatzzweck an ein Narkosegerät oder an eine Frischgasquelle unter Verwendung einer Gasdosiereinheit angeschlossen. Grundsätzlich ist man bestrebt, den Gehalt an Narkosegas im Operationssaal so gering wie möglich zu halten. Ein aus dem Stand der Technik bekanntes Beatmungsbesteck weist zu diesem Zweck einen Überbeutel auf, welcher den eigentlichen Beatmungsbeutel umgibt und mit einer Absaugung in Verbindung steht. Das Expirationsgas sowie überschüssige Narkosegas kann über eine im Beatmungsbeutel befindliche Öffnung in den Überbeutel gelangen und abgesaugt werden, ohne daß eine Belastung des Operationsraumes mit Narkosegas sich ergibt. Dieses bekannte Beatmungsbesteck hat jedoch mehrere gravierende Nachteile. Neben der manuell durchzuführenden Beatmung hat der Beatmungsbeutel die in gleicher Weise wichtige Funktion, daß der Anästhesist "am Beatmungsbeutel" die Lungenfunktion fühlen und hier ggf. unterstützend eingreifen kann. Dieses Fühlvermögen, d. h. die "Sensitivität" des Bestecks ist daher von entscheidender Bedeutung. Das Fühlvermögen wird durch die Verwendung des Überbeutels erheblich gestört, was dazu führt, daß in vielen Kliniken der Überbeutel nicht verwendet wird und das Narkosegas bzw. Expirationsgas daher in den Operationsraum ausströmt. Die Verwendung des Überbeutels ist auch insofern schwer handhabbar, als eine oftmals erwünschte Einstellung eines sog. positiven endexpiratorischen Drucks (PEEP) im System nur schwierig durchzuführen ist, da dies nämlich durch manuelles Verschließen der in dem Beatmungsbeutel vorgesehenen Öffnung geschehen muß, wodurch Expirationsgas sowie Narkosegas nicht in den Überbeutel abströmen kann. Demzufolge ist es auch schwierig, mit dem bekannten Besteck ein gleichbleibendes positives Druckniveau im System während der Atmung bzw. Beatmung, d. h. zwischen den Atemzyklen aufrechtzuerhalten.

Ein Ziel der vorliegenden Erfindung besteht daher darin, ein gattungsgemäßes Beatmungsbesteck zu schaffen, welches ein möglichst genaues Fühlen der Lungenfunktion (Resistance Compliance) des Patienten erlaubt, d. h. eine optimale Sensitivität besitzt und das Besteck von Expirationsgas bzw. Narkosegas entsorgt wird, ohne daß dieses in den Operationsraum einströmt und diesen belasten kann. Ferner soll das Beatmungsbesteck auf besonders einfache Weise handhabbar sein. Schließlich soll die Sicherheit des Patienten vor Druckerhöhungen während der Beatmung erhöht werden und die Effektivität der alveolären Ventilation erhöht werden.

Dieses Ziel wird erfindungsgemäß dadurch erreicht, daß

a) an dem Patienten abgewandten Ende des Expirationsgasschlauchs ein Ventil vorgesehen ist, welches den Expirationsgasschlauch mit einer Absaugung oder Entsorgung verbindet und die Aufrechterhaltung eines gewissen Druckniveaus im Beatmungskreis gewährleistet,

b) der Beatmungsbeutel über eine Verbindungseinrichtung zwischen Beatmungsmaske bzw. Endotrachealkatheter und Ventil in den Expirationsgasschlauch eingeschaltet ist.

Die Erfindung vermeidet jegliche Belastung des Operationsraumes mit Expirations- bzw. Narkosegas, wobei gleichzeitig ein äußerst sensitives, manuelles Fühlen der Lungenfunktion durch den Anästhesisten möglich ist. Neben dem Ventil befinden sich im System keine weiteren Ventile bzw. anderweitige Öffnungen, so daß die Lungenfunktion ausschließlich und unverfälscht am Beatmungsbeutel "gefühlt" werden kann. Die Erfindung ermöglicht zudem die Aufrechterhaltung eines positiven endexpiratorischen Druckniveaus. Die Beatmung kann daher bei erhöhtem Druckniveau durchgeführt werden, was für unterschiedliche Einsatzzwecke (insbesondere zur Erhöhung der Oberfläche der Lunge) gewünscht ist und insbesondere der Effektivität der alveolären Ventilation erhöht wird und ein Kollabieren von Alveolen verhindert wird.

Vorzugsweise ist das Ventil als insbesondere manuell betätigbares Feinregulierventil ausgebildet. Je nach Anwendung bzw. Patientenalter sowie Konstitution kann der Anästhesist mit einfachem Handgriff das Druckniveau im System wählen bzw. jederzeit während der Behandlung verändern.

Das Ventil bzw. Feinregulierventil ist in vorteilhafter Weise derart ausgestaltet, daß es mehrere einstellbare Druckbereiche aufweist, wobei die einzelnen Druckbereiche jeweils durch Lösen einer Sperre, welche am Ventil vorgesehen ist, zugänglich sind. Der Anästhesist kann daher je nach den Anforderungen in einem ganz bestimmten Druckbereich arbeiten, wobei eine versehentliche Druckerhöhung durch Überdrehen des Ventils in einen höheren Druckbereich wirksam ausgeschlossen werden kann.

Demgegenüber bleibt die Sperre beim Zurückdrehen des Ventils von einem höheren in den nächstniedrigeren Druckbereich unwirksam, d. h. die Sperre muß beim Zurückdrehen bei Änderung des jeweiligen Druckbereichs nicht manuell betätigt werden.

Zweckmäßigerweise ist am Feinregulierventil ein Druckbereich von 0 - 100 mbar einstellbar, dieser Druckbereich ist vorzugsweise im Stellbereich von 0 - 40 mbar, 40 - 60 mbar, 60 - 80 mbar, 80 - 100 mbar mit einer dazwischenliegenden Sperre unterteilt.

Zweckmäßigerweise gewährleistet das Ventil bzw. Feinregulierventil die Aufrechterhaltung eines gewissen Druckniveaus im Beatmungskreis (PEEP), wobei das Ventil bzw. Feinregulierventil als Sicherheitsventil arbeitet, wenn der betreffende Druckbereich überschritten wird. Eine Lungenschädigung durch erhöhten Druck während der Beatmung kann hierdurch ausgeschlossen werden.

Gemäß einer weiteren Ausgestaltung der vorliegenden Erfindung weist das Ventil bzw. Feinregulierventil einen Druckmesser mit Anzeige auf. Der Anästhesist hat hierdurch die Möglichkeit, ständig den Beamtungsdruck im System zu überwachen. Eine unbeabsichtigte Druckerhöhung in den Atmungswegen - aus welchen Gründen auch immer - kann schwerwiegende Folgen hervorrufen.

In vorteilhafter Weise ist der Druckmesser mit einem Alarmgeber verbunden.

Eine Ausgestaltung der Erfindung ist ferner dadurch gekennzeichnet, daß als Verbindungseinrichtung zum Zwischenschalten des Beamtungsbeutels ein T-Stück vorgesehen ist. Es wurde überraschenderweise gefunden, daß die Verwendung eines T-Stücks als Verbindungseinrichtung die "Sensitivität" des Beamtungsbeutels das manuelle Fühlen der Lungenfunktion betreffend, aufgrund der Anordnung in Kombination mit dem eingestellten Druck am Ventil während der Beatmung zusätzlich positiv beeinflußt. Der Beatmungsbeutel ist zudem durch die Verwendung des T-Stücks im Vergleich zu in den Expirationsgasschlauch integrierten Beatmungsbeutel wesentlich besser für den Anästhesisten handhabbar.

Die oben angesprochene Sensititvität des Beatmungsbeutels während der Beatmung läßt sich erstaunlicherweise noch dadurch verbessern, daß der mit dem Beatmungsbeutel in Verbindung stehende Schenkel des T-Stücks mit dem zum Patienten weisenden Schenkel einen stumpfen Winkel einnimmt.

Erfindungsgemäß wird weiterhin ein Koaxialschlauchsystem verwendet, derart, daß der Frischgasschlauch innerhalb des Expirationsgasschlauchs zur Beatmungsmaske bzw. zum Endotrachealkatheter geführt wird und die beiden Schlauchenden unmittelbar in die Beatmungsmaske bzw. in den Endotrachealkatheter münden. Die Schlauchanordnung bestehend aus Expirationsgasschlauch und innenliegendem Frischgasschlauch wirkt hierbei gleichsam als Wärmetauscher, wodurch das zugeführte Frischgas durch das wärmere Expirationsgas erwärmt wird, während es im System zur Beatmungsmaske hinströmt. Desweiteren wirkt bei dieser Ausgestaltung der Frischgasschlauch während der Handhabung des Beatmungsbestecks nicht störend. Eine Vermischung von Frischgas mit Expirationsgasmengen erfolgt daher ausschließlich an der Beatmungsmaske bzw. Tubus, nicht jedoch innerhalb des Beatmungsbestecks, d. h. innerhalb des Expirationsgasschlauches.

Zweckmäßigerweise ist das jeweils Patientenseitige Ende des Frischgasschlauchs und Expirationsgasschlauchs mit einem Koaxialstück verbunden, welches einen mittigen Durchlaß (Frischgasöffnung) sowie seitliche, um die mittige Öffnung herum angeordnete Durchlässe (Expirationsgasöffnungen) aufweist. Hierdurch wird eine mittige Zufuhr des Frischgases unmittelbar in die Beatmungsmaske bzw. den Endotrachealkatheter erzielt, wobei Expirationsgas gleichsam ringförmig in den Expirationsgasschlauch abströmen kann.

Dadurch, daß der mittige Durchlaß des Koaxialstücks innerhalb des Koaxialstücks möglichst nahe an die Beatmungsmaske bzw. den Endrotrachaelkatheter heranreicht, wird der Totraum wirksam verringert, in dem Frischgas mit Expirationsgas vor dem Einatmen vermischt werden kann.

Weiterhin kann das Ventil bzw. Feinregulierventil ausgangsseitig zweckmäßigerweise mit einem Reservoirbeutel in Verbindung stehen, welcher als Puffer zwischen Beatmungsbesteck und der Absaugung bzw. Entsorgung dient.

Eine aufwendige Sterilisierung wird vermieden, wenn die Schläuche einschließlich der Verbindungselemente als Einwegartikel ausgebildet und keimfrei verpackt sind.

Alternativ hierzu können die Verbindungselemente jedoch auch aus Metall und die Schläuche aus einem sterilisierbaren Stoff bestehen, um diese Teile gegebenenfalls in einem Autoklaven zu sterilisieren.

Zweckmäßigerweise kann unmittelbar vor dem Ventil bzw. Feinregulierventil ein Bakterienfilter eingeschaltet sein, so daß lediglich das Patientensystem keimfrei gehalten werden muß.

Die Erfinder des Beatmungsbestecks haben weiterhin gefunden, daß die Sensitivität des Beatmungsbeutels für den Anästhesisten dann überraschenderweise am besten ist, wenn sich der Beatmungsbeutel relativ nah an der Beatmungsmaske oder am Endotrachealkatheter befindet. Die Nähe des Beatmungsbeutels zur Beatmungsmaske bzw. Endotrachealkatheters findet ihre Grenze lediglich in der zu gewährleistenden Handhabbarkeit des Systems am Patienten.

Durch die Länge de Beatmungsmaske und Beatmungsbeutel verbindenden Schlauches kann die partielle Rückatmung des Patienten bestimmt werden. Gleichzeitig kann diese partielle Rückatmung durch die Höhe des Frischgasflows zusätzlich beeinflußt werden.

Versuche haben gezeigt, daß eine optimale Sensitivität dann zu erreichen ist, wenn die Länge des zwischen Beatmungsmaske bzw. Endotrachealkatheter und Beatmungsbeutel befindlichen Expirationsgasschlauches im Bereich von 20 - 40cm, vorzugsweise 25 - 35cm liegt.

Eine zweckmäßige Ausgestaltung des erfindungsgemäßen Beatmungsbestecks wird anhand der Zeichnungen im folgenden erläutert:

Es zeigen:

Fig. 1   Eine Ausgestaltung des erfindungsgemäßen Beatmungsbestecks in vereinfachter, schematisierter Darstellungsweise;

Fig. 2   das an der Beatmungsmaske angeordnete Koaxialstück in Schnittdarstellung in Seitenansicht (Fig. 2a) sowie aus der Blickrichtung A (Fig. 2b)

Fig. 3   das am Ventil-seitigen Ende des Beatmungsbestecks angeordnete Verbindungsstück in Schnittdarstellung;

Fig. 4   das T-Stück zur Verbindung des Beatmungsbeutels mit dem Expirationsgasschlauch in Schnittdarstellung;

Fig. 5   eine schematisierte, stark vereinfachte Darstellung des im erfindungsgemäßen Beatmungsbesteck verwendeten Feinregulierventils;

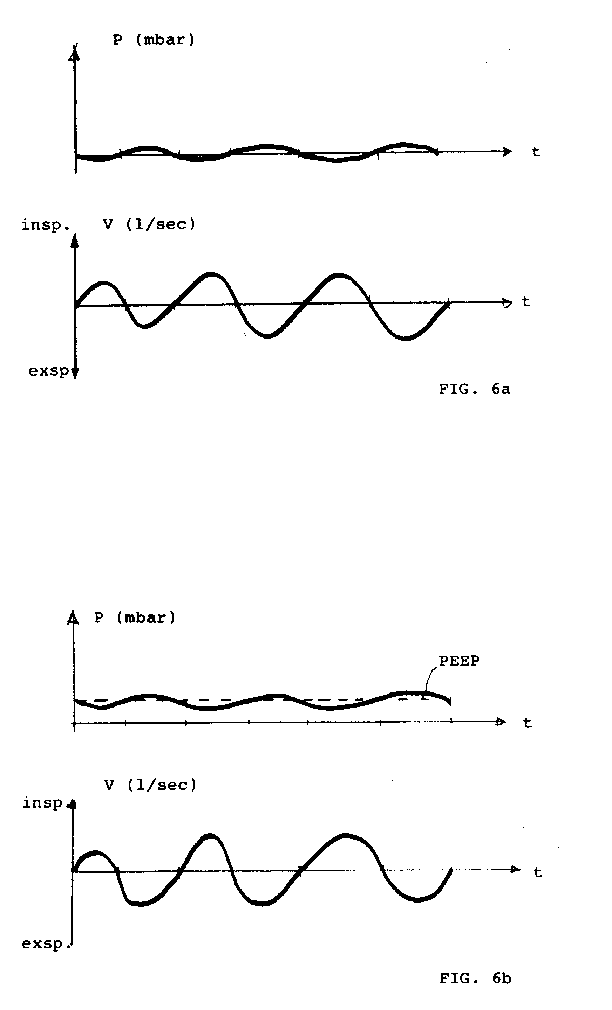

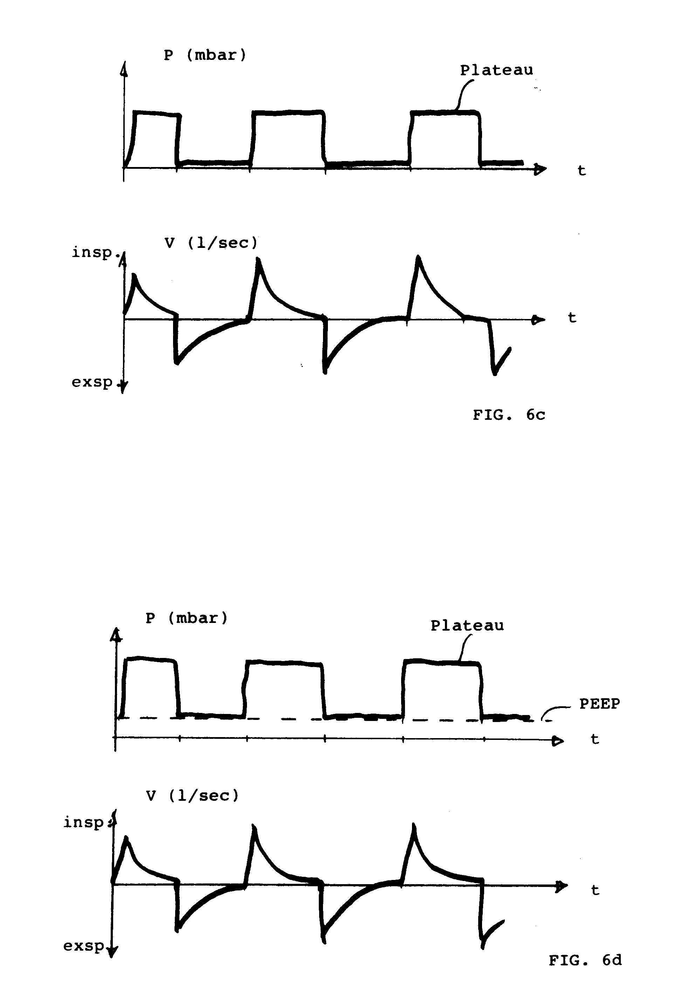

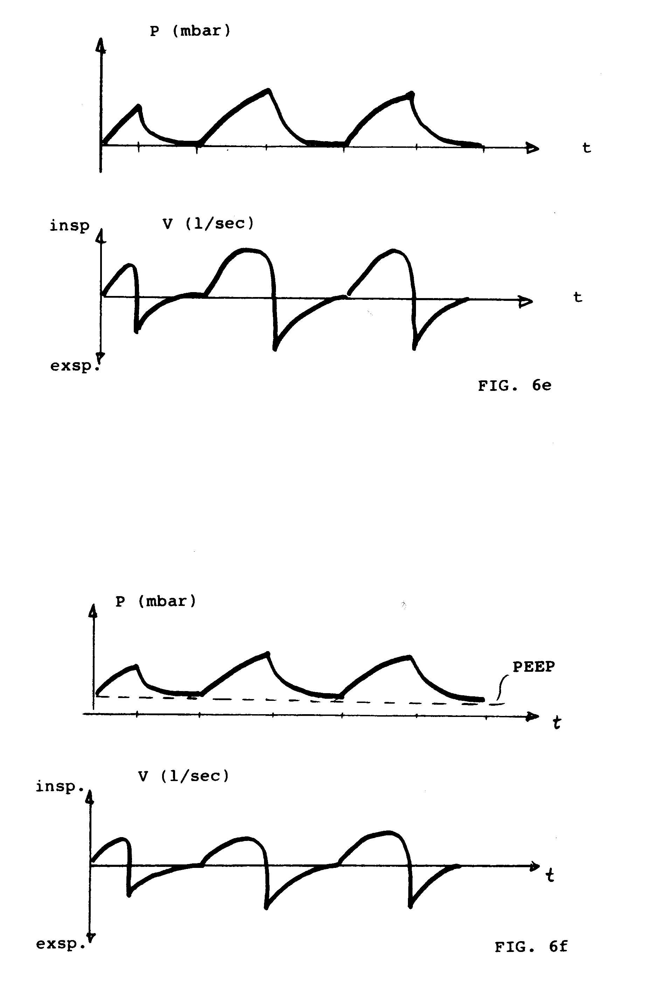

Fig. 6   den Druckverlauf (obere Kurve) sowie den Gasvolumenstrom während der Inspiration und Expiration jeweils über der Zeit aufgetragen bei einer Spontanatmung ohne PEEP (Fig. 6a), den entsprechenden Druckverlauf sowie die Gasvolumenmengen bei Spontanatmung mit PEEP (Fig. 6b), den Druckverlauf sowie die Gasvolumenströmung bei einer manuell kontrollierten Beatmung mit Plateau (Fig. 6c), den Druckverlauf sowie die Gasvolumenströmung gemäß Fig. 6c jedoch mit PEEP, den Druckverlauf sowie die Gasvolumenströmung bei manuell kontrollierter Beatmung ohne PEEP (Fig. 6e) sowie der Druckverlauf und die Gasvolumenströmung gemäß der unter Fig. 6e gezeigten Beatmung jedoch mit PEEP (Fig. 6f).

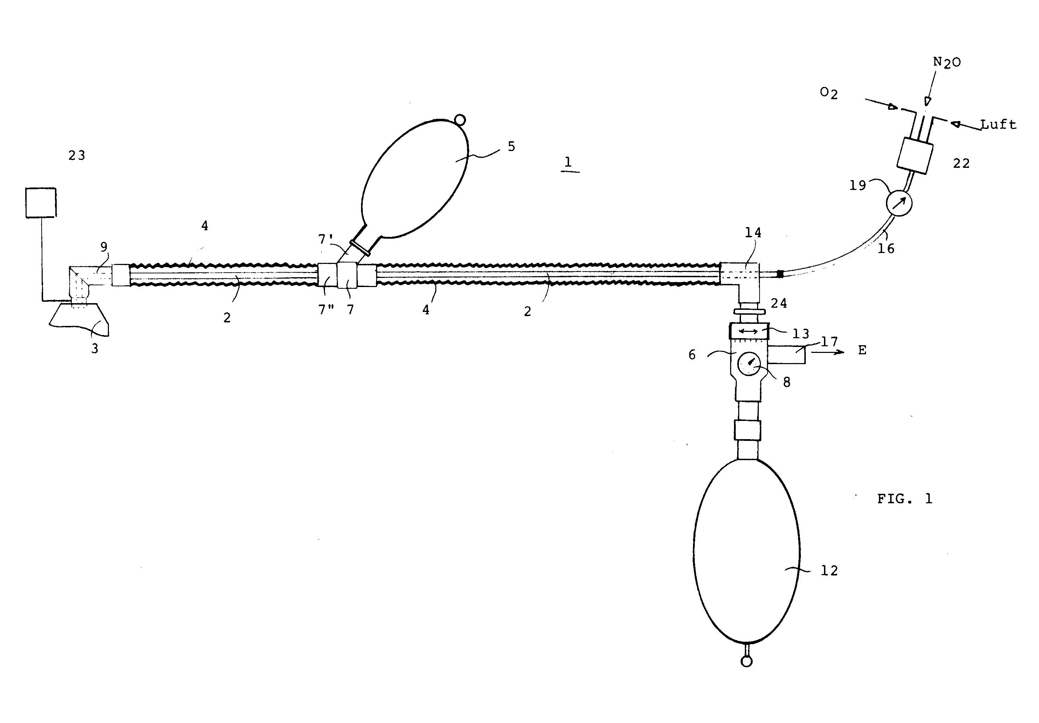

Bezugsziffer 1 in Fig. 1 bezeichnet das erfindungsgemäße Beatmungsbesteck in seiner Gesamtheit. Das Beatmungsbesteck 1 umfaßt einen sog. "Koaxialschlauch", welcher aus einem Expirationsgasschlauch 4 sowie einem innerhalb des Expirationsgasschlauchs 4 geführten Frischgasschlauch 2 besteht. Der Frischgasschlauch 2 steht über ein Verbindungsstück 14 mit einer Frischgaszufuhr 16 in Verbindung. Die Regelung der Frischgaszufuhr bzw. Narkosegaszufuhr erfolgt zweckmäßigerweise über ein Durchflußmeter 19. Mit dem Durchflußmeter 19 steht eine Dosiereinheit 22 in Verbindung, mittels welcher das Narkosegas dosiert werden kann. Diese Dosiereinheit 22 ist zweckmäßigerweise als bewegliches Teil ausgebildet und daher als Bestandteil des Narkosebestecks 1 ausgelegt. Hierdurch wird gleichsam eine Art Komplettsystem geschaffen.

Der Frischgasschlauch 2 sowie Expirationsgasschlauch 4 verlaufen koaxial zu einem Koaxialstück 9, welches unmittelbar mit der Beatmungsmaske 3 verbunden ist, wobei das Koaxialstück dafür sorgt, daß das Frischgas (bzw. Narkosegas) den Frischgasschlauch erst in der Beatmungsmaske 3 verläßt. Hierdurch wird vermieden, daß bereits im Beatmungsbesteck 1 eine Vermischung des zugeführten Narkosegases mit Expirationsgasmengen und damit eine Rückatmung von $CO_2$ erfolgt.

Zur Erhöhung der wirksamen Oberfläche und hierdurch erreichten verbesserten Wärmeaustauscheignung ist der Expirationsgasschlauch 4 als sog. "Faltenschlauch" ausgebildet.

In den Expirationsgasschlauch 4 ist möglichst nahe an der Beatmungsmaske 3 ein T-Stück 7 zur Einschaltung eines Beatmungsbeutels 5 vorgesehen. Die Nähe des Beatmungsbeutels 5 zur Beatmungsmaske 3 wird begrenzt durch die Handhabbarkeit des Beatmungsbestecks 1 am Patienten. Der Beatmungsbeutel 5 soll jedoch möglichst nahe an der Beatmungsmaske 3 liegen, da hierbei eine besonders gute Sensitivität der Lungenfunktion durch den Anästhesisten erfolgen kann. Eine optimale Sensitivität des Beatmungsbeutels wird bei der beschriebenen Anordnung in einem Abstand von 20 - 40 cm, vorzugsweise 25 - 35 cm erzielt. In diesem Bereich hat der Anästhesist aufgrund des Zusammenwirkens der die Erfindung kennzeichnenden Merkmale ein optimales manuelles Gefühl für die Lungenfunktion des zu behandelnden Patienten.

Das T-Stück 7 ist zweckmäßigerweise derart geformt, daß der zur Beatmungsmaske 3 hinweisende Schenkel 7'' des T-Stücks 7 relativ zum Schenkel 7', welcher zum Beatmungsbeutel hinführt einen stumpfen Winkel aufweist. Hierdurch wird in überraschender Weise die Sensitivität betreffend das manuelle Fühlen der Lungenfunktion durch den Anästhesisten positiv beeinflußt.

Die Verwendung eines T-Stücks bewirkt ferner eine besonders günstige Handhabbarkeit, da der Beatmungsbeutel 5 gleichsam parallelgeschaltet ist, so daß im Vergleich zu einer "in Serie Schaltung" der Frischgasschlauch, welcher von der Frischgaszufuhr 16 kommt, nicht störend wirkt. Die Verbindung der Expirationsgasschlauchabschnitte mit dem T-Stück 7 bzw. mit dem Verbindungsstück 14 sowie mit dem Koaxialstück 9 erfolgt durch einfaches Ineinanderschieben bzw. Aufeinanderstecken. Der Expirationsgasschlauch weist hierfür geeignete (nicht dargestellte) Steckhülsen auf.

Der Frischgasschlauch 2 läuft im Bereich des T-Stücks 7 durch dieses mittig hindurch, im Beatmungsbeutel 5 befindet sich folglich lediglich Expirationsgas bzw. unverbrauchtes, während des Ausatemvorganges in den Expirationsgasschlauch gelangtes Narkosegas.

Das Beatmungsbesteck 1 ist ferner mit einem Ventil 6 , welches als Feinregulierventil ausgebildet ist, versehen. Das Ventil 6 steht über einen Bakterienfilter 24 mit dem Verbindungsstück 14 in Verbindung, welches das Ende des Expirationsgasschlauches 4 aufnimmt. Das Zwischenschalten eines Bakterienfilters 24 bewirkt eine Bakteriensperre, so daß keine Bakterien vom Ventil 6 in den Beatmungskreis gelangen können. Demzufolge kann der Faltenschlauch einschließlich der damit zusammenhängenden Teile vorteilhafterweise ausgetauscht werden, ohne daß jeweils eine Sterilisierung des Ventils 6 erfolgen müßte.

Das Ventil 6 gewährleistet über einen Regulierhahn 13 die Einstellung eines Druckniveaus innerhalb eines Druckbereiches, wobei dieser Druckbereich vorzugsweise im Bereich von 0 - 100 mbar einstellbar sein soll. Die Überwachung des im System, d. h. im Beatmungskreislauf, zu dem auch der Atemweg des Patienten gehört, vorherrschenden Beatmungsdrucks kann über einen Druckmesser 8 mit Anzeige vom Anästhesisten während der Behandlung jederzeit überwacht und bei Bedarf der Druck geändert werden. Zweckmäßigerweise sind über das Ventil mehrere voneinander getrennte Druckbereiche einstellbar, wobei durch manuelles Betätigen einer (nicht dargestellten) vorzugsweise manuell betätigbaren Sperre von dem einen in den nächsthöheren Stellbereich übergegangen werden kann. Der Anästhesist wählt hierbei zunächst den Druckbereich und stellt unter Zuhilfenahme des Regulierhahns 13 das gewünschte Druckniveau ein.

Die Beatmung kann hierdurch bei einem sog. positiven endexpiratorischen Druck (PEEP) innerhalb des betreffenden Druckbereichs erfolgen. Das Ventil 6 wirkt hierbei gleichzeitig als Sicherheitsventil und öffnet bei Erreichen der oberen Grenze des eingestellten Druckbereichs, um eine Schädigung der Lungenfunktion des Patienten zu vermeiden (vgl. hierzu auch die Druckplateaus in den Fig. 6c und 6d). Eine derartige Schädigung droht insbesondere bei Hustenanfällen des Patienten oder dergleichen, wodurch eine plötzliche Druckerhöhung im Beatmungssystem hervorgerufen wird.

Das Ventil 6 steht über einen Ausgang 17 mit einer mit E gekennzeichneten Entsorgung (Narkosegasentsorgung nach ISO-Standard) in Verbindung, welche beispielsweise aus einer Absaugung unter Zwischenschaltung einer Absorbereinrichtung bestehen kann. An der Unterseite des Ventils 6 befindet sich ein Reservoirbeutel 12, welcher während des Betriebs des Beatmungsbestecks 1 stets gefüllt sein sollte.

Der Sinn und Zweck des Reservoirbeutels 12 liegt darin, ein Reservoir für die Absaugung zu bilden und hierdurch zu vermeiden, daß die Absaugung unmittelbar auf das Beatmungsbesteck 1 einwirkt. Der Reservoirbeutel 12 hat auch die Aufgabe, überschüssiges, von dem Beatmungsstück 1 stammendes Gas zu sammeln, sozusagen gleichsam als Puffer zwischen Beatmungsbesteck 1 und Entsorgung E zu wirken.

Anstelle der am Koaxialstück 9 angeordneten Beamtungsmaske 3 kann in äquivalenter Weise ebenfalls ein Endotrachealkatheter vorgesehen sein, welcher dem Patienten direkt in die Atemwege eingeführt wird.

Wie bereits eingangs erwähnt, weist die Frischgaszuführung 16, über die Sauerstoff, ein Sauerstoffluftgemisch oder Narkosegas zugeführt wird, ein regelbares Durchlußmeter 19 auf. Das System gemäß Fig. 1 umfasst folglich zwei Hauptvariable, nämlich einmal den Betrag der Frischgaszufuhr, regelbar über das Durchflußmeter 19 sowie den im System vorherrschenden Druck, regelbar über das Ventil 6.

Am Koaxialstück 9 befindet sich ein $CO_2$-Sensor 23, mittels welchem überwacht werden kann, ob eine Rückatmung von Expirationsgasmengen, welche $CO_2$ enthalten, stattfindet. Die Zufuhr an Frischgas bzw. Narkosegas über die Frischgaszufuhr 16 wird über das Durchflußmeter 19 regelmäßig derart eingestellt, daß Patienten-seitig während der Einatmung sich genügend Frischgas bzw. Narkosegas auch im Expirationsgasweg befindet, so daß der Patient kein Expirationsgas rückatmet. Dies wird durch eine bestimmte Durchflußmenge an Frischgas bzw. Narkosegas erreicht mit der Folge, daß sich nach jedem Expirationsvorgang ein Frischgas bzw. Narkosegaspolster zwischen Beatmungsmaske und Expirationsgasmenge bildet. Wird die Zufuhr von Frischgas bzw. Narkosegas über das Durchflußmeter 19 reduziert, wird dieses Frischgaspolster geringer. In manchen Fällen kann es notwendig sein, eine Rückatmung von Expirationsgasmengen bewußt hervorzurufen, beispielsweise dann, wenn die Atmungsaktivität des Patienten auf diese Weise stimuliert werden soll.

Je höher die Frischgaszufuhr eingestellt ist, desto niedriger ist der $CO_2$-Wert d. h. die Rückatmung, da vergleichsweise mehr unverbrauchtes Frischgas das System verläßt.

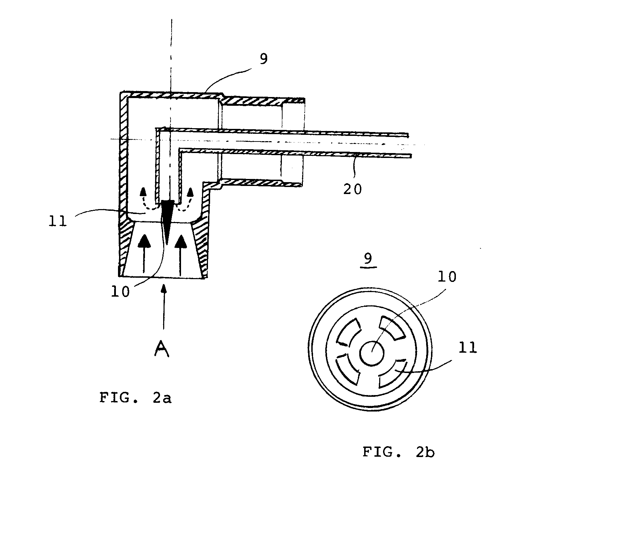

Fig. 2a und b zeigen das Koaxialstück 9, welches unmittelbar mit der Beatmungsmaske 3 in Verbindung steht. Das Koaxialstück 9 umfasst eine mittige Frischgaszuführung 20, auf die der Frischgasschlauch 2 aufgesteckt ist. Am Ende dieser Zuführung 20 besitzt das Koaxialstück 9 einen mittigen Durchlaß 10 bzw. Auslaß in Richtung zur Beatmungsmaske 3. Um diesen mittigen Durchlaß 10 herum (vgl. Fig. 2b) sind mehrere seitliche Durchlässe 11 vorgesehen, welche dafür sorgen, daß das Expirationsgas ringförmig in den Expirationsgasschlauch 4 einströmen kann. Die in Fig. 2 dargestellten Pfeile sollen die entsprechenden Strömungen kennzeichnen. Die punktiert gekennzeichneten Pfeile stellen den Verlauf des Frischgasstromes dar, während der Patient ausatmet.

Hierbei gelangt der Frischgasstrom unmittelbar nach dem mittigen Durchlaß 10 in die seitlichen Durchlässe 11 aufgrund der Gegenströmung der Expirationsgasmenge.

Während der Beatmung des Patienten kann - wie bereits oben erwähnt - durch Einstellung der Frischgaszufuhr 16 der Betrag an rückgeatmetem Expirationsgas eingestellt werden. Je höher die Frischgaszufuhr eingestellt ist, desto weniger atmet der Patient das Expirationsgas zurück, welches sich bereits im Expirationsgasschlauch 4 befindet. Die genaue Einstellung der erforderlichen Menge erfolgt in der bereits eingangs beschriebenen Weise über die Messung des $CO_2$-Werts des Gases, welches das Beatumungsbesteck 1 über das Ventil 6 verläßt.

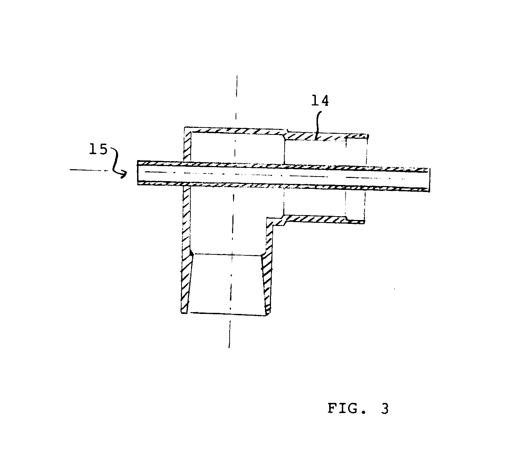

In Fig. 3 ist das Verbindungsstück 14 dargestellt, welches zur Einleitung des Frischgases über den Frischgasanschluß 15 in das aus Frischgasschlauch 2 und Expirationsgasschlauch 4 bestehende Koaxialschlauchsystem dient. Die untere Öffnung des Verbindungsstücks 14 steht unmittelbar mit dem Ventil in Verbindung.

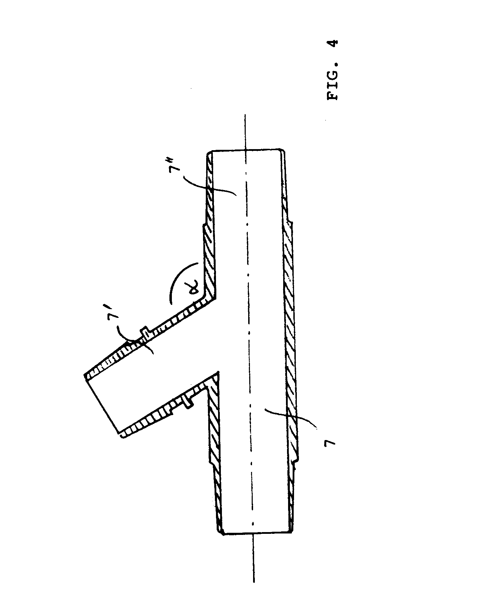

Fig. 4 zeigt das T-Stück zur Verbindung des Beatmungsbeutels 5 mit dem Expirationsgasschlauch 4. Zweckmäßigerweise weist der Schenkel 7' des T-Stücks, welcher zum Beatmungsbeutel 5 verläuft, zum Schenkel 7'', welcher zur Beatmungsmaske 3 hin gerichtet ist, einen stumpfen Winkel $\alpha$ auf.

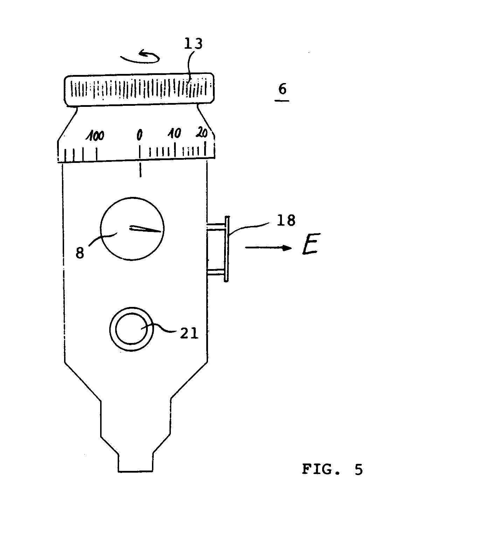

Fig. 5 beschreibt das Ventil 6, welches zur Aufrechterhaltung eines gewissen Überdrucks im Beatmungsbesteck 1 dient. Das Ventil weist einen mit dem Expirationsgasschlauch 4 verbundenen Einlaß 21 auf, über den das Expirationsgas einem Manometer bzw. Beatmungsdruckmesser mit Anzeige 8 zugeführt wird. Das im Beatmungssystem vorherrschende Druckniveau kann über einen Regulierhahn 13, welcher mit einer entsprechenden Beschriftung versehen ist, in einfacher Weise manuell eingestellt werden. Das Ventil 6 wirkt - wie bereits oben erwähnt - gleichzeitig als Sicherheitsventil, so daß sich der Beatmungsdruck bei eingestelltem Druckniveau innerhalb eines vorgegebenen Druckbereichs bewegen kann. Das Ventil 6 ist beispielsweise über einen Flansch 18 mit (nicht dargestellten) Entsorgungsleitungen bzw. Entsorgungsschläuchen verbunden. Die Entsorgung des Expirationsgases bzw. Narkosegases erfolgt in der üblichen Weise.

In den Figuren 6a - 6f sind dem besseren Verständnis halber unterschiedliche Beatmungskurven dargestellt. Die obere Kurve betrifft jeweils die sog. Druckkurve, d. h. den Druckverlauf innerhalb des Beatmungssystems in Abhängigkeit der Zeit. Die jeweils unmittelbar darunter dargestellte Kurve zeigt das Gasvolumen d. h. den Gasvolumendurchsatz bei der Beatmung während der Inspiration und Expiration ebenfalls in Abhängigkeit der Zeit. Bei der in Fig. 6a dargestellten Spontanatmung entsteht bei der Inspiration ein geringfügiger Unterdruck mit der Folge, daß Luftvolumen inspiriert wird. Der Druckbereich ändert sich bei der Spontatmung im Vergleich zum Umgebungsdruck nur geringfügig.

Durch Veränung des Drucks im Beatmungssystem durch Verstellung des Ventils 6 kann der positive endexpiratorische Druck (PEEP) eingestellt werden, mit der Folge, daß - wie aus Fig. 6b ersichtlich ist - die spontane Atmung im Positiven Druckbereich durchgeführt wird. An den Volumendurchsatzmengen des Atmungsgases bzw. der Atmungsluft während der Inspiration bzw. Expiration ändert sich hierbei nichts. Die Einstellung des Positiven endexpiratorischen Drucks (PEEP) bewirkt eine Erhöhung der alveolären Ventilation bzw. ein Verhindern eines Kollabierens von Alveolen.

Fig. 6c zeigt die Verhältnisse bei manuell kontrollierter Beatmung mit einem sog. Druckplateau, welches durch den Einsatz eines Sicherheitsventils, welches bei einem bestimmten Druckwert automatisch öffnet, auftritt. Das Sicherheitsventil soll in erster Linie eine unerwünschte Druckerhöhung im System beispielsweise durch einen Hustenanfall des Patienten oder dergleichen vermeiden.

Die mit der Erfindung mögliche Kombination von Druckplateau und positiven endexpiratorischen Druck während der Beatmung ist in Figur 6d dargestellt. Die Beatmung wird ausschließlich bei einem positiven endexpiratorischen Druck durchgeführt, wobei gleichzeitig eine schädliche Drucküberhöhung im Beatmungssystem vermieden werden kann. Die Figuren 6e sowie 6f zeigen die Druckverhältnisse und Strömungsverhältnisse bei manuell kontrollierter Beatmung ohne bzw. mit einem positiven endexpiratorischen Druck (PEEP) jedoch ohne Einsatz eines sog. Überdruckventils. In den Fällen der Figuren 6c bis 6f wird die Beatmung aktiv über den Beatmungsbeutel durchgeführt, was an dem erhöhten Druckanstieg während der Beatmung zu erkennen ist. Im Gegensatz dazu atmet der Patient bei der Spontanatmung gemäß den Figuren 6a und 6b selbst, d. h. ohne Unterstützung durch manuelle Betätigung des Beatmungsbeutels oder einer vergleichbaren maschinellen Einrichtung.

Die vorliegende Erfindung bietet aufgrund der kombinatorischen Wirkung der einzelnen Merkmale eine besonders hohe Handhabbarkeit bei gleichzeitig optimierter Sensitivität betreffend das manuelle Fühlen der Lungenfunktion des Patienten über den Beatmungsbeutel durch den Anästhesisten. Darüber hinaus erfolgt keinerlei Belastung des Operationsraumes mit Narkosegas, wobei gleichzeitig die Patientensicherheit durch Vermeidung von unerwünschten, schädlichen hohen Drücken wesntlich erhöht wird. Die Erfindung stellt daher eine außergewöhnliche Bereicherung auf dem Gebiet der Anästhesietechnik dar.

**Patentansprüche**

1.  Beatmungsbesteck zur manuellen oder maschinellen Beatmung oder spontanen Atmung, insbesondere Narkosebeatmung mit einem Schlauch zur Zuführung von Gasen, insbesondere Narkosegasen (Frischgasschlauch), einem Schlauch, vorzugsweise Faltenschlauch zur Abführung von Expirationsgasmengen und ggf. nicht eingeatmetem Gas (Expirationsgasschlauch), einem Beatmungsbeutel, welcher im Expirationsgasweg angeordnet ist, einer Beatmungsmaske oder Endotrachealkatheter oder - tubus sowie einem Verbindungsstück zur Verbindung von Frischgasschlauch und Expirationsgasschlauch an der Beatmungmaske bzw. am Endotrachealkatheter,

    dadurch gekennzeichnet,

    daß
    a) an dem dem Patienten abgewandten Ende des Expirationsgasschlauchs (4) ein Ventil (6) vorgesehen ist, welches den Expirationsgasschlauch mit einer Absaugung oder Entsorgung verbindet und die Aufrechterhaltung eines gewissen Druckniveaus im Beatmungskreis gewährleistet,
    b) der Beatmungsbeutel (5) über eine Verbindungseinrichtung zwischen Beatmungsmaske (3) bzw. Endotrachealkatheter und Ventil (6) in den Expirationsgasschlauch (4) eingeschaltet ist.

2.  Beatmungsbesteck nach Anspruch 1,

    dadurch gekennzeichnet,

    daß das Ventil (6) ein vorzugsweise manuell betätigbares Feinregulierventil ist.

3.  Beatmungsbesteck nach Anspruch 1 oder 2,

    dadurch gekennzeichnet,

    daß das Ventil (6) bzw. Feinregulierventil mehrere einstellbare Druckbereiche aufweist, die jeweils durch Lösen einer Sperre zugänglich sind.

4.  Beatmungsbesteck nach Anspruch 3,

    dadurch gekennzeichnet,

    daß Ventil (6) bzw. Feinregulierventil eine einzige Stellschraube oder einen einzigen Regulierhahn (13) aufweist und als Sperre ein von Hand betätigbarer Mechanismus vorgesehen ist, welcher die Verstellbarkeit der Stellschraube bzw. des Regulierhahns (13) in mehrere Stellbereiche aufteilt, wobei durch manuelles Betätigen des Mechanismus von dem einen in den nächsthöheren Stellbereich übergegangen werden kann.

5.  Beatmungsbesteck nach Anspruch 3 oder 4,

    dadurch gekennzeichnet,

    daß die Sperre bzw. der Mechanismus beim Zurückdrehen der Stellschraube bzw. des Regulierhahns (13) von einem höheren Stellbereich zu einem nächstniedrigeren Stellbereich den Übergang nicht verhindert, d. h. nicht manuell betätigt zu werden braucht.

6.  Beatmungsbesteck nach den Ansprüchen 2 - 5,

    dadurch gekennzeichnet,

    daß durch das Feinregulierventil ein Druckbereich von 0 - 100 mbar einstellbar ist.

7.  Beatmungsbesteck nach Anspruch 6,

    dadurch gekennzeichnet,

    daß Stellbereiche von 0 - 40 mbar, 40 - 60 mbar, 60 - 80 mbar, 80 - 100mbar mit einer dazwischenliegenden Sperre vorgesehen sind.

8.  Beatmungsbesteck nach den Ansprüchen 1 - 7,

    dadurch gekennzeichnet,

    daß das Ventil (6) bzw. Feinregulierventil die Aufrechterhaltung eines gewissen Drucks im Beatmungskreis innerhalb eines Druckbereichs gewährleistet und das Ventil (6) bzw. Feinregulierventil als Sicherheitsventil arbeitet, wenn der betreffende Druckbereich überschritten wird.

9.  Beatmungsbesteck nach den Ansprüchen 1 - 8,

    dadurch gekennzeichnet,

    daß das Ventil (6) bzw. Feinregulierventil einen Druckmesser (8) mit Anzeige und/oder Alarmgeber aufweist.

10. Beatmungsbesteck nach den Ansprüchen 1 - 9,

    dadurch gekennzeichnet,

    daß als Verbindungseinrichtung zum Zwischenschalten des Beatmungsbeutels (5) ein T-Stück (7) vorgesehen ist.

11. Beatmungsbesteck nach Anspruch 10,

    dadurch gekennzeichnet,

    daß der mit dem Beatmungsbeutel (5) in Verbindung stehende Schenkel (7') des T-Stücks (7) mit dem zum Patienten weisenden Schenkel (7'') einen stumpfen Winkel einnimmt.

12. Beatmungsbesteck nach Anspruch 10 oder 11,

    dadurch gekennzeichnet,

    daß zwischen T-Stück (7) und Beatmungsbeutel (5) ein Verbindungsschlauch vorgesehen ist.

13. Beatmungsbesteck nach den Ansprüchen 1 - 12,

    dadurch gekennzeichnet,

    daß ein Koaxialschlauchsystem verwendet wird, derart, daß der Frischgasschlauch (2) innerhalb des Expirationsgasschlauchs (4) zur Beatmungsmaske (3) bzw. zum Endotrachealkatheter geführt wird und die beiden Schlauchenden unmittelbar in die Beatmungsmaske (3) bzw. in den Endotrachealkatheter münden.

14. Beatmungsbesteck nach Anspruch 13,

    dadurch gekennzeichnet,

    daß die beiden Schlauchenden mit einem Koaxialstück (9) verbunden sind, welches einen mittigen Durchlaß (10) (Frischgasöffnung) sowie seitliche, um den mittigen Durchlaß (10) herum angeordnete Durchlässe (11) (Expirationsgasöffnungen) aufweist.

15. Beatmungsbesteck nach Anspruch 14

    dadurch gekennzeichnet,

    daß der mittige Durchlaß (10) des Koaxialstücks (9) innerhalb des Koaxialstücks (9) möglichst nahe an die Beatmungsmaske (3) bzw. den Endotrachealkatheter heranreicht.

16. Beatmungsbesteck nach den Ansprüchen 1 - 15,

    dadurch gekennzeichnet,

    daß das Ventil (6) bzw. Feinregulierventil ausgangsseitig unmittelbar mit einem Reservoirbeutel (12) in Verbindung steht.

17. Beatmungsbesteck nach den Ansprüchen 1 - 16,

    dadurch gekennzeichnet,

    daß die Verbindungselemente und Verbindungseinrichtungen aus Metall oder autoklavierbarem Kunststoff oder Gummi und die Schläuche aus sterilisierbarem, insbesondere autoklavierbarem Kunststoff oder Gummi bestehen.

18. Beatmungsbesteck nach den Ansprüchen 1 - 17,

    dadurch gekennzeichnet,

    daß die Schläuche einschließlich der Verbindungselemente bzw. Verbindungseinrichtungen als Einwegsystem vorgesehen sind.

19. Beatmungsbesteck nach den Ansprüchen 1 - 18,

    dadurch gekennzeichnet,

daß sich der Beatmungsbeutel (5) relativ nahe an der Beatmungsmaske (3) oder am Endotrachealkatheter befindet.

20. Beatmungsbesteck nach Anspruch 19,

    dadurch gekennzeichnet,

daß die Länge des zwischen Beatmungsmaske (3) oder Endotrachealkatheter und Beatmungsbeutel (5) befindlichen Expirationsgasschlauches (4) im Bereich von 20 bis 40cm, vorzugsweise 25 bis 35cm liegt.

21. Beatmungsbesteck nach den Ansprüchen 1 - 19,

    dadurch gekennzeichnet

daß das Beatmungsbesteck eine integrierte Gasdosiereinheit (22) sowie ein nachgeschaltetes Durchflußmeter (19) aufweist.

22. Beatmungsbesteck nach den Ansprüchen 1 - 21,

    dadurch gekennzeichnet,

daß am Koaxialstück (9), vorzugsweise zwischen Beatmungsmaske (3) bzw. Endotrachealkatheter und Koaxialstück (9) ein $CO_2$-Sensor (23) eingeschaltet ist.

FIG. 1

FIG. 2a

FIG. 2b

14

15

FIG. 3

FIG. 4

FIG. 5

FIG. 6a

FIG. 6b

P (mbar)

Plateau

t

V (l/sec)

insp.

exsp.

t

FIG. 6c

P (mbar)

Plateau

PEEP

t

V (l/sec)

insp.

exsp.

t

FIG. 6d

P (mbar)

t

insp

V (l/sec)

exsp.

FIG. 6e

P (mbar)

PEEP

t

insp.

V (l/sec)

exsp.

FIG. 6f

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | GB-A-2 160 429 (BERESFORD WEST)<br>* Zusammenfassung; Abbildungen 4,5 * * Seite 1, Zeile 70 - Zeile 111 *<br>– – – | 1-3,13,16 | A 61 M 16/08 |
| Y | WO-A-9 003 820 (HENKIN ET AL.)<br>* Zusammenfassung; Abbildungen 2-4 * * Seite 11, Zeile 21 - Seite 12, Zeile 27 *<br>– – – | 1-3,13,16 | |
| A | GB-A-2 095 121 (MILLER)<br>* Zusammenfassung; Abbildungen 1-3 * * Seite 1, Zeile 72 - Zeile 121 * * Seite 2, Zeile 38 - Zeile 68 *<br>– – – | 1 | |
| A | WO-A-8 001 044 (FISCHER)<br>* Seite 7, Zeile 27 - Seite 8, Zeile 29; Abbildungen 5,6 *<br>– – – | 1,21 | |
| A | GB-A-2 073 028 (PORTER INSTRUMENT CO.)<br>* Zusammenfassung; Abbildung 6 * * Seite 1, Zeile 117 - Seite 2, Zeile 16 *<br>– – – – – | 1 | |

| RECHERCHIERTE SACHGEBIETE (Int. Cl.5) |
|---|
| A 61 M |

Der vorliegende Recherchenbericht wurde für alle Patentansprüche erstellt

| Recherchenort | Abschlußdatum der Recherche | Prüfer |
|---|---|---|
| Den Haag | 04 Oktober 91 | ZEINSTRA H.S.J.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

                                                                

& : Mitglied der gleichen Patentfamilie, übereinstimmendes Dokument